(19) Europäisches Patentamt / European Patent Office / Office européen des brevets

(11) **EP 3 040 033 A1**

(12) **EUROPEAN PATENT APPLICATION**
published in accordance with Art. 153(4) EPC

(43) Date of publication:
**06.07.2016 Bulletin 2016/27**

(21) Application number: **14841190.3**

(22) Date of filing: **23.07.2014**

(51) Int Cl.:
**A61B 8/08** *(2006.01)*

(86) International application number:
**PCT/JP2014/069484**

(87) International publication number:
**WO 2015/029651 (05.03.2015 Gazette 2015/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **26.08.2013 JP 2013174828**

(71) Applicant: **Hitachi Aloka Medical, Ltd.
Tokyo 181-8622 (JP)**

(72) Inventors:
• **ASAMI, Rei
Tokyo 100-8280 (JP)**
• **YOSHIKAWA, Hideki
Tokyo 100-8280 (JP)**
• **TABARU, Marie
Tokyo 100-8280 (JP)**

(74) Representative: **MERH-IP Matias Erny Reichl Hoffmann
Patentanwälte PartG mbB
Paul-Heyse-Strasse 29
80336 München (DE)**

(54) **DIAGNOSTIC ULTRASOUND APPARATUS AND ELASTICITY EVALUATION METHOD**

(57) Disclosed is a technique capable of reducing deterioration of measurement accuracy and reproducibility due to a long measurement time and acquiring an ultrasound image with high diagnostic performance in measurement of a shear wave velocity of radiation pressure elastography. In the radiation pressure elastography, information relating to a motion (fluctuation) in a measurement region is extracted while detecting a shear wave from echo signals due to irradiation of tracking pulses, and is provided to a user as reliability information indicating the reliability of a measurement result. Further, a factor of the fluctuation is specified from the extracted information, and is presented to the user. Furthermore, when arithmetically averaging plural times of measurement results, weighting is performed using the reliability information.

Fig.1

**Description**

TECHNICAL FIELD

The present invention relates to an ultrasound imaging technique that noninvasively acquires information about the inside of a subject using ultrasound, and more particularly, to an elastography technique that images the hardness of a tissue.

BACKGROUND ART

A diagnostic ultrasound apparatus is a medical imaging apparatus that applies ultrasound to the body from the outside of the body and images a signal reflected from the inside of the body according to an elapsed time and the intensity of the signal. Since the ultrasound has a property of being reflected according to the Snell's law on an interface where acoustic impedances become different, by visualizing a difference between the acoustic impedances which are delicately different from each other depending on tissues in the body, it is possible to draw a structure of the tissues.

There is an elastography technique that images the hardness of a tissue, instead of the structure of the tissue, using a diagnostic ultrasound apparatus. The hardness of the tissue has a close relationship with a lesion, and brings important information for diagnosis. As such an elastography technique, there is a radiation pressure elastography that generates a shear wave and measures a shear wave velocity from displacement generated by propagation of the shear wave to obtain the hardness of a tissue. Assuming that the Poisson's ratio of the tissue is calculated as 0.5 and a compressive wave velocity is sufficiently larger than a transverse wave velocity, a Young' s modulus E which is an index of the hardness is simply expressed as the following Expression (1).

$$E = 3\rho V_S^2 \quad \cdot \quad \cdot \quad \cdot \quad (1)$$

Here, p represents density, and Vs represents a shear wave velocity. An absolute value of the hardness is obtained from the shear wave velocity using Expression (1).

The shear wave is generated by emitting focused ultrasound to a single point and applying radiation pressure to a tissue. Pulses applied pulses here are referred to as radiation pressure generating pulses (push pulses). Displacement of the shear wave generated by the push pulses is detected by shear wave detecting pulses (tracking pulses).

Since the absolute value of the hardness is calculated in the radiation pressure elastography, it is necessary to measure the displacement due to the shear wave with high accuracy, and to calculate a shear wave velocity with high reproducibility. In order to enhance the reproducibility, there is a technique that measures plural shear wave velocities at plural positions in a measurement region by one-time measurement and presents an average of the obtained measurement values as a measured value (for example, see PTL 1). In the technique disclosed in PTL 1, the measured value is evaluated by the size of a value deviated from the measured value, and is presented together with the evaluation result. According to this technique, it is considered that the effect of the deviated value in arithmetic averaging is suppressed to be small, and thus, the measurement accuracy is enhanced.

CITATION LIST

PATENT LITERATURE

PTL 1: US-A-2010/0016718

SUMMARY OF INVENTION

However, as described above, in the radiation pressure elastography, two types of ultrasound pulses of the push pulses and the tracking pulses are applied. In the technique disclosed in PTL 1, since the two types of pulses are repeatedly applied, it takes long time for measurement. Accordingly, a deviation of an imaging surface due to a body motion based on breathing, heart beating or the like of a subject, or shaking of a user' s hand, or the like occurs, which makes deterioration of the measurement accuracy and reproducibility.

Specifically, first, it may be considered that a deviation occurs in a measurement range and a portion different from a measurement portion is measured due to the above-mentioned motion. Further, even in a minor deviation, it may be considered that an original tense of the detected shear wave and a measured tense thereof are deviated from each other and a detected shear wave velocity is deviated from an original propagation velocity. In addition, even when a surface deviation due to, particularly, the body motion or the like does not occur, it may be considered that "the degree of compression (distortion)" of the liver due to a heart rate, for example, is changed according to tenses. Here, the degree of compression affects the shear wave velocity, which may deteriorate the measurement accuracy.

Considering the above problems, an object of the invention is to provide a technique capable of reducing deterioration of measurement accuracy and reproducibility due to a long measurement time in measurement of a shear wave velocity in a radiation pressure elastography, and acquiring an ultrasound image with high diagnostic performance.

In radiation pressure elastography, according to the invention, information relating to a motion (fluctuation) in a measurement region is extracted while detecting a shear wave from echo signals due to irradiation of tracking pulses and the extracted information is provided to a user as reliability information indicating the reliability of a measurement result. Further, a factor of the fluctuation is specified from the extracted information, and is presented to the user. In addition, when arithmetically averaging plural times of measurement results, weighting is performed using the reliability information.

According to the invention, in radiation pressure elastography, it is possible to reduce deterioration of measurement accuracy and reproducibility due to a long measurement time, and to acquire an ultrasound image with high diagnostic performance.

## BRIEF DESCRIPTION OF DRAWINGS

Fig. 1 is a block diagram illustrating a diagnostic ultrasound apparatus according to an embodiment of the invention.
Fig. 2(a) is a diagram illustrating a B-mode image example according to an embodiment of the invention, and 2 (b) is an enlarged view of a measurement region (region 220 in Fig. 2(a)) according to the embodiment of the invention.
Fig. 3 is a diagram illustrating a depth-directional change of a correlation coefficient in the measurement region according to the embodiment of the invention.
Fig. 4 (a) is a diagram illustrating a B-mode image example of an imaging region according to the embodiment of the invention, and Figs. 4 (b) to 4 (d) are diagrams illustrating changes of correlation coefficients due to a non-shear wave fluctuation.
Fig. 5 is a diagram illustrating a display screen example according to the embodiment of the invention.
Figs. 6(a) to 6(d) are diagrams illustrating display screen examples according to the embodiment of the invention.
Fig. 7 is a flowchart illustrating an imaging process according to the embodiment of the invention.
Fig. 8 is a block diagram illustrating a diagnostic ultrasound apparatus according to a modification example of the embodiment of the invention.
Figs. 9(a) to 9(c) are diagrams illustrating instructions received from a user according to the modification example of the embodiment of the invention.
Fig. 10 is a flowchart illustrating processes after display according to the modification example of the embodiment of the invention.

## DESCRIPTION OF EMBODIMENTS

Hereinafter, examples of embodiments of the invention will be described with reference to the accompanying drawings. In the entire drawings for description of the respective embodiments, the same names and the same reference numerals are given to the same functional components as long as there is no particular mention, and description thereof will not be repeated. Further, in this description, a shear wave velocity represents a propagation velocity of a shear wave. In the respective embodiments of the invention, for example, it is possible to perform evaluation with respect to information relating to tissue characterization such as distortion, Young's modulus, viscosity, or volume elasticity.

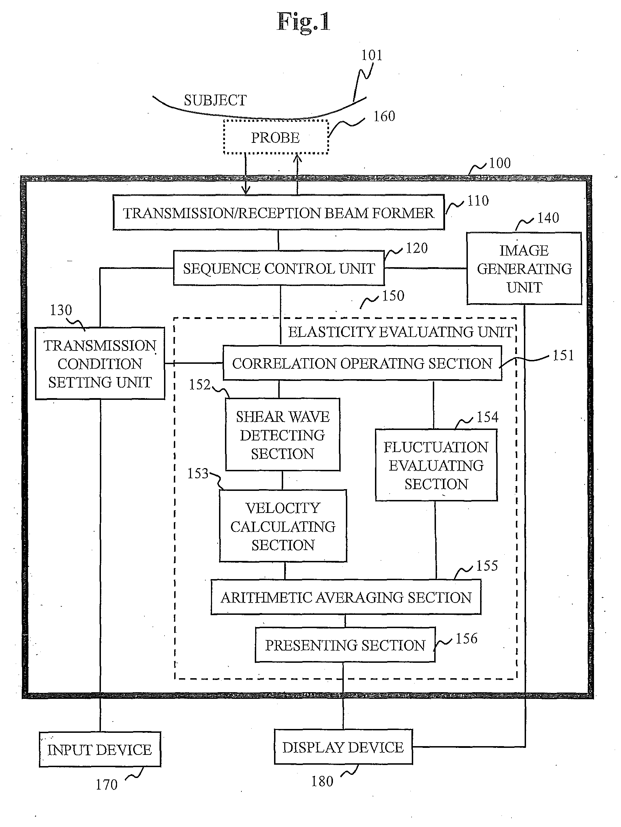

First, a diagnostic ultrasound apparatus 100 of an embodiment will be described. Fig. 1 is a block diagram illustrating the diagnostic ultrasound apparatus 100 according to this embodiment.

The diagnostic ultrasound apparatus 100 of this embodiment employs a radiation pressure elastography technique that performs measurement for applying (transmitting) a radiation pressure to a measurement region of a subject and transmitting focusing burst ultrasound (hereinafter, referred to as push pulses) for generating a shear wave and pulse ultrasound (hereinafter, referred to as tracking pulses) for detecting propagation of the shear wave generated by the transmission of the push pulses, and obtains a propagation velocity of the shear wave as a property of a tissue in the measurement region. Further, in order to enhance reliability and reproducibility, the measurement is repeated, and the obtained results are arithmetically averaged.

Here, the diagnostic ultrasound apparatus 100 of this embodiment extracts information relating to a motion (fluctuation) in the measurement region from echo signals of the tracking pulses, and presents the extracted information to a user as an index relating to the reliability of information obtained by the radiation pressure elastography. Further, a factor of the fluctuation is specified from the extracted information relating to the fluctuation, and is presented to the user. In addition, the information relating to the fluctuation is also used for weighting in the arithmetic averaging.

The diagnostic ultrasound apparatus 100 of this embodiment includes a transmission/reception beam former 110, a sequence control unit 120, a transmission condition setting unit 130, an image generating unit 140, and an elasticity evaluating unit 150. Further, a probe 160, an input device 170, and a display device 180 are connected to the diagnostic ultrasound apparatus 100.

<Transmission beam former>

The transmission/reception beam former 110 transmits a transmission beam to the probe 160 according to an instruction from the sequence control unit 120, and receives an echo signal received by the probe 160.

Specifically, an electric signal of ultrasound pulses transmitted from each element of the probe 160 is generated. The generated electric signal is converted into an analog signal by a D/A converter provided in the transmission beam former, and then, is transmitted to the probe 160 to then be applied to a subject. A signal reflected from an interface where acoustic impedances become different in the course of propagating in the subject is received by the probe 160 as a reception echo signal, is converted into a digital signal through a process reverse to the transmission process., The digital signal is subject to addition processing such as phasing addition, is subject to a process such as decay compensation, and then, is converted into complex RF data.

<Sequence control unit>

The sequence control unit 120 determines a timing when an ultrasound pulse is transmitted, a timing when an echo signal is received, characteristics of the ultrasound pulse to be transmitted, and the like, according to imaging conditions set through the transmission condition setting unit 130, as a pulse sequence. Further, the sequence control unit 120 controls the transmission/reception beam former 110 and executes measurement, according to the determined pulse sequence. In this embodiment, the radiation pressure elastography is executed. Thus, the sequence control unit 120 of this embodiment generates the pulse sequence to execute measurement for performing transmission of the push pulses, repetitive transmission of the plural tracking pulses, and reception of the echo signals based on the tracking pulses.

<Transmission condition setting unit>

The transmission condition setting unit 130 sets, according to a position where a shear wave is generated (hereinafter, referred to as a measurement region), received from the user, transmission conditions of the push pulses in the measurement region and transmission conditions of the tracking pulses for detecting the shear wave generated in the region. The transmission conditions to be set include acoustic pressure parameters such as a focusing position, a transmission angle, a burst length, a voltage, a frequency, and a transmission opening.

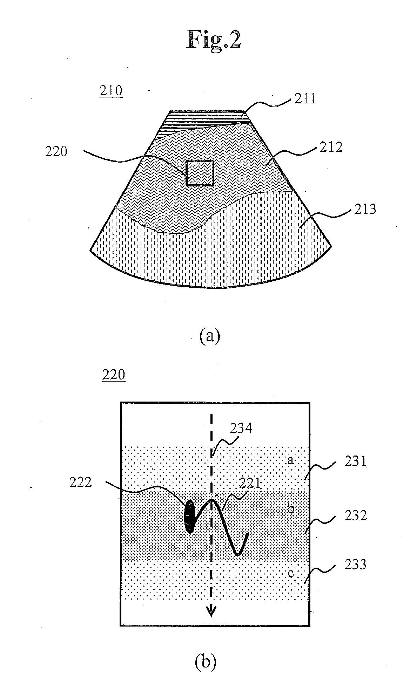

Figs. 2(a) and 2(b) are diagrams illustrating concepts of push pulses and tracking pulses. Fig. 2(a) is an example of a B-mode image 210, and Fig. 2(b) is an enlarged view of a measurement region 220 in the B-mode image 210. An arrow 234 represents a depth direction.

A shear wave 221 based on a radiation pressure, generated at a focus 222 of push pulses in the measurement region 220 propagate in a tissue. The tracking pulses are transmitted for detection of the shear wave. Thus, the tracking pulses are continuously transmitted during a propagation time of the shear wave at the shortest, with respect to one-time push pulse.

The transmission conditions of the push pulses are set so that the push pulses are transmitted to a desired position 222 in the designated measurement region 220, and the transmission conditions of the tracking pulses are set so that the shear wave 221 generated by the push pulsesis measured by echo signals thereof. Further, with respect to the tracking pulses, the number of times of transmission in one-time measurement, the number of times of repetition, transmission positions of plural tracking pulses for one-time repetition, and the like are set as the transmission conditions.

<Image generating unit>

The image generating unit 140 receives the complex RF data obtained by the transmission/reception beam former 110 under the control of the sequence control unit 120, and generates a tomographic image. The image generating unit 140 plots a luminance value of RF data obtained from one echo signal (beam) in a depth direction according to a reception time. The image generating unit 140 arranges the plotted luminance values with respect to plural beams in a device array of longitudinal direction of the probe 160 to accumulate two-dimensional information, and generates a tomographic image from the accumulated information. The generated tomographic image is displayed on the display device 180.

For example, in the B-mode imaging in which the intensity of the echo signal is imaged as a luminance, the number of beams in the longitudinal direction of the probe 160 affects an imaging frame rate. In order to secure real-time performance, normally, several tens to several hundreds of beams are used to acquire a single B-mode image.

<Probe>

The probe 160 may be a probe 160 capable of transmitting and receiving a sequence for the above-described shear wave measurement, and preferably, may employ a 1D array probe of a linear, convex, or sector shape, or a 1.5-dimensional or a two-dimensional array probe for three-dimensional imaging, or the like.

<Elasticity evaluating unit>

The elasticity evaluating unit 150 obtains information about the hardness of a tissue of the measurement region 220. In this embodiment, the elasticity evaluating unit 150 detects a shear wave generated by transmission of push pulses and obtains its velocity (shear wave velocity), to thereby obtain information indicating the hardness of the tissue. The shear wave velocity is calculated from displacement generated by propagation of the shear wave. Further, the elasticity evaluating unit 150 of this embodiment calculates information (reliability information) indicating the reliability of the obtained shear wave velocity, and presents the result to a user.

To implement the above, the elasticity evaluating unit 150 of this embodiment includes a correlation operating section 151, a shear wave detecting section 152, a velocity calculating section 153, a fluctuation evaluating section 154, an arithmetic averaging section 155, and a presenting section 156, as shown in Fig. 1.

<Correlation operating section>

The correlation operating section 151 performs a correlation operation in a time direction with respect to RF data obtained from a received echo signal. In this embodiment, since the RF data is complex RF data, a complex correlation operation is performed. The complex correlation operation may be performed between pieces of RF data which are temporally adjacent to each other. Alternatively, after reference RF data is determined, and then, the complex correlation operation may be performed between the reference RF data other pieces of RF data.

<Shear wave detecting section>

The shear wave detecting section 152 detects a shear wave generated at a focus of push pulses by transmission of burst ultrasound (push pulses) focused on a subject 101 using received echo signals obtained by repeatedly transmitting plural shear wave detection pulses (tracking pulses). In this embodiment, the shear wave detecting section 152 detects a peak of the shear wave from the complex correlation result in the correlation operating section 151, and obtains a detection position and a detection time. In this embodiment, before detection of the peak, an optimal filtering process is performed with respect to the complex correlation result.

<Velocity calculating section>

The velocity calculating section 153 calculates a shear wave velocity which is a propagation velocity of a shear wave. In this embodiment, the shear wave velocity is calculated by a detection time of a peak of the shear wave, a detection position thereof, and a shear wave generation position. Specifically, the shear wave velocity is calculated from a focus of push pulses, and a transmission position of tracking pulses where the peak of the shear wave is observed.

<Fluctuation evaluating section>

The fluctuation evaluating section 154 evaluates a fluctuation in the measurement region 220 including a propagation region of a shear wave, and obtains the evaluation result as reliability information indicating the reliability of the shear wave velocity. The fluctuation of a target to be evaluated is a fluctuation that affects measurement accuracy of radiation pressure elastography and reproducibility. In this embodiment, first, a region where the fluctuation is to be detected is specified in the measurement region 220, and a fluctuation of a tissue at a predetermined position (evaluation position) in the specified region is evaluated.

First, a method for specifying the region where the fluctuation is to be detected will be described.

Two types of fluctuations may be considered as fluctuations that affect the measurement accuracy of the radiation pressure elastography or the reproducibility. One type of fluctuation is a surface deviation caused when the probe 160 held by a user moves, and the other type of fluctuation is a deviation of a measurement portion due to a periodical body motion of a subject such as heart beating or breathing. Hereinafter, the former is referred to as fluctuation caused from a practitioner, and the latter is referred to as fluctuation caused from a body motion.

It should be noted that the shear wave of a measurement target in the radiation pressure elastography is obtained by measuring a weak motion of a tissue. Accordingly, it is necessary to distinguish the motion due to the shear wave of the measurement target from the fluctuation that affects the measurement accuracy or the reproducibility, and to detect only the fluctuation. Hereinafter, the former motion will be referred to as a shear wave fluctuation, and the latter fluctuation will be referred to as a non-shear wave fluctuation. The fluctuation evaluating section 154 of this embodiment specifies a region which is not affected by the shear wave fluctuation, sets a predetermined position in the specified region as an evaluation position, and evaluates a non-shear wave fluctuation at the evaluation position.

Here, the position which is not affected by the shear wave fluctuation will be described. As described above, Fig. 2 (a) is a conceptual view of the ultrasound image (B-mode image) 210, and Fig. 2 (b) is an enlarged view of the measurement region 220 in Fig. 2(a).

As shown in Fig. 2(a), in an ultrasound field of view, the tissues are displayed in a layer structure. Here, a three-layer structure of a layer 211, a layer 212, and a layer 213 is shown as an example. Further, as shown in Fig. 2(b), the shear wave 221 propagates in a lateral direction from a portion (shear wave generating position) 222 where a radiation pressure is generated. That is, the shear wave 221 propagates only within a predetermined range (in the figure, a region b232 which will be hereinafter referred to as a shear wave propagation region) in a depth direction (downward direction in the figure). Accordingly, it may be considered that a region a231, a region c233, or the like in the figure, other than the shear wave propagation region 232, is a position which is not affected by the motion of the shear wave. These regions are referred to as non-propagation regions 231 and 233.

The fluctuation evaluating section 154 of this embodiment calculates reliability information based on a fluctuation at a predetermined position (evaluation position) in depth regions (non-propagation regions 231 and 233) having a depth different from the depth of a depth region (shear wave propagation region 232) where the shear wave propagates, in the measurement region 220. The evaluation position is set to a position close to the propagation generating position as much as possible, in the non-propagation regions 231 and 233.

The fluctuation evaluating section 154 determines the shear wave propagation region 232 and specifies the non-propagation regions 231 and 233 using the detection result of the shear wave detecting section 152. The shear wave propagation region 232 is specified by a generation position of the shear wave and the amplitude of the shear wave. The generation position of the shear wave 221 is a position where the radiation pressure is generated by the push pulses. This position is a focus depth determined by a transmission opening width calculated from the number of elements used in generation of the push pulses, and the depth of a focus. Further, the amplitude of the shear wave 221 is specified by a distance between the position of the peak of the shear wave detected by the shear wave detecting section 152, and the focus depth.

Further, the shear wave propagation region 232 may be independently specified without using the detection result of the shear wave detecting section 152. For example, the shear wave propagation region 232 may use the complex correlation operation result in the correlation operating section 151. That is, the shear wave propagation region 232 may be specified using a correlation coefficient.

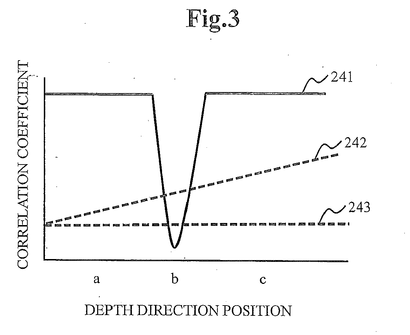

Generally, at a position where the motion (fluctuation) is present, the correlation coefficient is reduced regardless of the type of the motion such as a shear wave fluctuation or a non-shear wave fluctuation. Here, as described above, the shear wave 221 is generated at a limited position in the depth direction, for example, only in the shear wave propagation region 232 in Fig. 2(b). Accordingly, with respect to the change of the correlation coefficient in the depth direction, as schematically shown in Fig. 3, the reduction of the correlation coefficient is present locally in a change 241 of the correlation coefficient due to the shear wave. On the other hand, changes 242 and 243 of the correlation coefficient due to the non-shear wave fluctuation, that is, due to a surface deviation of the probe 160 or a body motion are uniform regardless of the regions in Fig. 2 (b). A complex correlation operation result that is actually obtained is a composite of the changes 241, 242, and 243 of the correlation coefficient.

A region where the correlation coefficient is locally reduced is detected using the above changes, and the detected region is set as the shear wave propagation region 232. The region where the correlation coefficient is locally reduced is detected by a differential operation or the like, for example. After determination of the shear wave propagation region 232, a method for specifying the non-propagation regions 231 and 233, and a method for determining the evaluation position are performed in the same way as in the above description.

The fluctuation evaluating section 154 of this embodiment calculates, as reliability information, a fluctuation index KM obtained by indexing the degree (size of the fluctuation) of the fluctuation of the position (evaluation position) specified by the above method. Generally, the correlation coefficient of the correlation operation is greatly reduced as the motion is larger. In this embodiment, using such a relationship, an average value of formalized correlation coefficients at the evaluation position is set as the fluctuation index KM, for example. In this case, the value of the fluctuation index KM becomes small as the fluctuation becomes large. The correlation operation used herein may be a correlation operation which is common to that in the shear wave detection, or may be a different correlation operation.

As described above, in this embodiment, at plural positions in the measurement region 220, plural times of measurement are performed, and plural shear wave velocities are obtained. Further, in each measurement, the fluctuation index KM is calculated. Here, the fluctuation evaluating section 154 may further calculate dispersion with respect to an average value of the respective fluctuation indexes KM obtained in the plural times of measurement, for example, a standard deviation as the reliability information. Further, the fluctuation evaluating section 154 may calculate a standard deviation of the shear wave velocities obtained in the respective measurements as the reliability information.

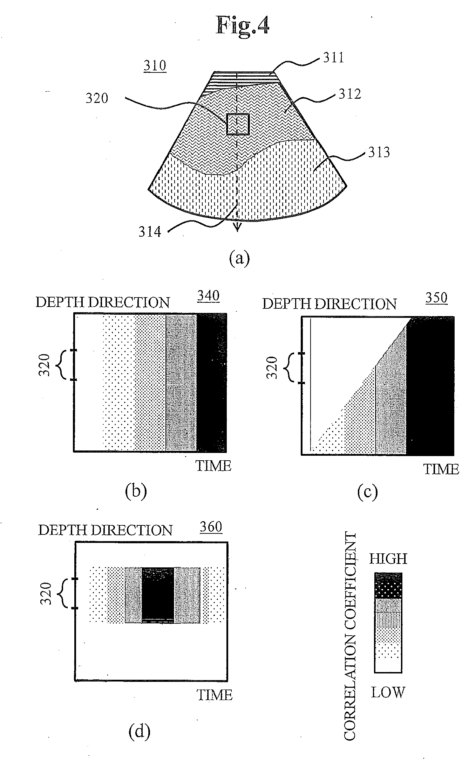

Next, a method for specifying a factor of the fluctuation detected by the shear wave detecting section 152, by the fluctuation evaluating section 154, will be described. Here, as the factor, whether the fluctuation is caused from the body motion or from a practitioner in a maintenance procedure or the like of the probe 160 is specified. The specification is performed by identifying a change pattern of the correlation coefficient. The specification method will be described with reference to Figs. 4 (a) to 4(d). Fig. 4 (a) is a diagram illustrating a B-mode image 310. Figs. 4(b) to 4(d) are diagrams illustrating change patterns of a complex correlation coefficient of tracking pulses due to a non-shear wave fluctuation in an imaging region.

For example, when imaging the liver, as shown in Fig. 4 (a), on the B-mode image 310, a superficial tissue 311 such as skin, muscle or fat is observed in front of the liver 312, and another tissue 313 such as a digestive tract separated by the diaphragm is observed inside the liver 312. Arrow 314 represents the depth direction.

When the fluctuation is caused from a practitioner, there are two types of factors including side slip of the probe 160 and deviation thereof in a rotation direction. The side slip of the probe 160 occurs when an installation position of the probe 160 is deviated. Further, the deviation thereof in the rotation direction occurs when the installation position of the probe 160 is not deviated but an angle thereof is deviated.

A correlation coefficient change pattern 340 in the case of the side slide (deviation of the installation position of the probe 160) is shown in Fig. 4 (b). As shown in the figure, in the case of the side slip, reduction of the correlation coefficient simultaneously occurs in the entire regions of the imaging surface, including the outside of the measurement region 320.

A correlation coefficient change pattern 350 when the probe 160 is deviated in the rotation direction is shown in Fig. 4(c). As shown in the figure, in this case, reduction of the correlation coefficient occurs in the entire regions of the imaging surface, including the outside of the measurement region 320. However, in this case, timings when the correlation coefficient is reduced are not the same time, and in a deeper portion, that is, at a position distant from the surface of the probe 160, the reduction is early started.

In contrast, the fluctuation due to a periodic body motion such as heart beating or breathing is changed according to tissues. Specifically, while the superficial tissue 311 such as a skin or a fat layer or another tissue 313 which is disposed inside the liver separated by the diaphragm does not move, an internal organ such as the liver 312 disposed in the middle portion shows a characteristic and periodic motion. Thus, as shown in Fig. 4(d), a correlation coefficient change pattern 360 in this case shows a periodic change only at the position of the internal organ (liver 312) in the middle portion, including the measurement region 320.

The fluctuation evaluating section 154 of this embodiment detects the change patterns of the correlation coefficient, and determines whether the fluctuation is caused from the practitioner or the periodic body motion. For example, a reference pattern which is a reference of the changes or information for specifying the reference pattern may be retained in advance in a storage device provided in the diagnostic ultrasound apparatus 100, and the fluctuation evaluating section 154 may compare a detected pattern with the reference pattern to perform determination of the factor of the fluctuation.

The determination result is presented to a user through the presenting section 156. Further, here, as the factor, when the fluctuation is caused from the practitioner, a message for prompting re-measurement may be displayed. In addition, when the above-described standard deviation of the fluctuation index KM is equal to or greater than a predetermined threshold value, it is determined that the measurement is not appropriate, and similarly, a message for prompting re-measurement may be displayed.

It is preferable that a signal used when the fluctuation evaluating section 154 evaluates the fluctuation is the above-described complex correlation operation result obtained by transmitting the tracking pulses. However, the signal is not limited thereto. For example, data obtained by the B-mode imaging may be used.

<Arithmetic averaging section>

The arithmetic averaging section 155 calculates an average value of plural shear wave velocities obtained by plural times of measurement at plural positions in the measurement region 220.

In this embodiment, here, the fluctuation index KM may be used for weighting. That is, shear wave velocities obtained through respective measurements have different contributions to average value calculation according to their reliabilities (here, fluctuation index KM). Thus, the reliability of the obtained arithmetic average velocity is enhanced.

A weighted average is calculated by the following Expression (2), for example.

$$Vs_{mean} = \frac{1}{n} \sum_{i=1}^{n} \frac{Vs_i \, KM_i}{KM_{mean}} \quad \cdots \quad (2)$$

Here, n represents the number of times of measurement (n is an integer of 2 or more), $Vs_i$ represents a shear wave velocity obtained by i-th measurement, $KM_i$ represents a fluctuation index obtained by the i-th measurement, $KM_{mean}$ represents an average value of n fluctuation indexes KMi, $Vs_{mean}$ represents an arithmetic average velocity obtained by a weighted arithmetic average.

<Presenting section>

The presenting section 156 presents a shear wave velocity Vs for each measurement calculated by the velocity calculating section 153, an arithmetic average velocity $Vs_{mean}$ calculated by the arithmetic averaging section 155, reliability information, and the like to the user. In this embodiment, display information to be displayed in the display device 180 is generated using the measurement results and calculation results. The display information may be a numerical value, or may be a qualitative graph or a color map display.

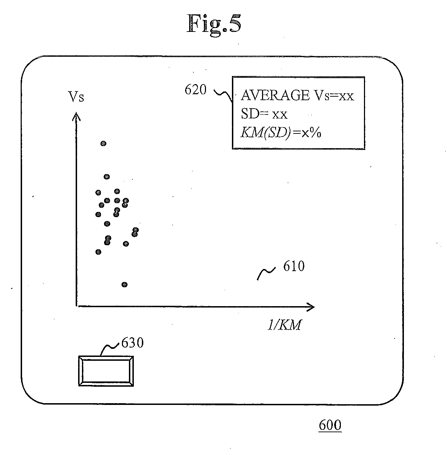

Fig. 5 shows an example of a screen created by the presenting section 156 as display information. On a display screen 600, a scatter plot 610 of the shear wave velocity Vs for each measurement and a reciprocal (1/KM) of a fluctuation index, and reference information 620 are displayed.

The scatter plot 610 is obtained by plotting measurement results on a graph where the shear wave velocity Vs and the reciprocal (1/KM) of the fluctuation index KM are used as respective axes.

In the reference information 620, the arithmetic averaging velocity $V_{mean}$ calculated by the arithmetic averaging section 155, a standard deviation SD of the shear wave velocities and a standard deviation KM (SD) of the fluctuation indexes KM calculated by the fluctuation evaluating section 154, and the like are displayed. Here, it is preferable that the display of the fluctuation index KM has a form such that the standard deviation of KM is displayed by the percentage thereof with respect to the average value, but the display may be performed using a different statistic and an absolute value.

The display screen 600 may be configured so that a reception button 630 that receives an instruction for re-measurement is displayed.

Hereinafter, a specific display example will be described.

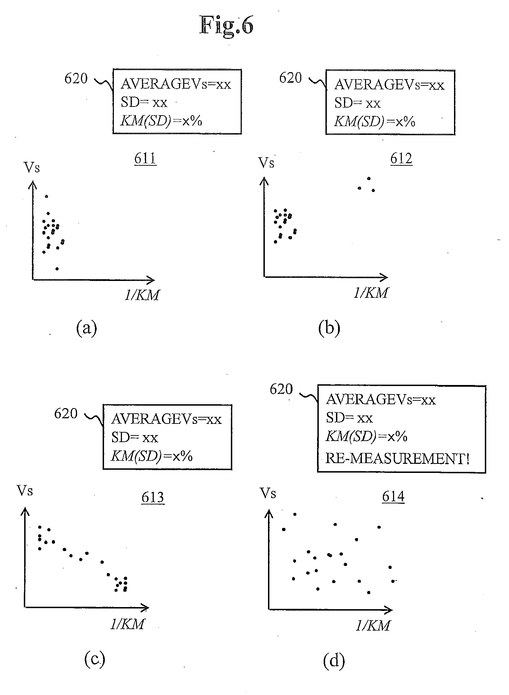

Fig. 6(a) is an example of a display screen 611 when a fluctuation in an imaging region is small. When excellent imaging is performed with little motion, the reciprocal of the fluctuation index KM is relatively small, and plotted points are collectively found in a range where the reciprocal of the fluctuation index KM is small.

Fig. 6(b) shows an example of a display screen 612 expected to be obtained when an abnormal value is present due to a certain motion. Two sets of plotted points are found. Since the reciprocal of the fluctuation index KM is large in a set in which the number of the plotted points is small, it is possible to suggest to a user that the shear wave velocity has been obtained when the motion is large.

.Fig. 6(c) shows an example of a display screen 613 expected to be obtained when there is a periodic motion. The example shows that plotted points are divided into two groups according to the size of the motion.

Fig. 6(d) shows an example of a display screen 614 obtained when the value of the reciprocal of the fluctuation index KM is large and measurement is not appropriate. In such a case, the presenting section 156 may be configured so that a message for prompting re-measurement is displayed together.

The diagnostic ultrasound apparatus 100 of this embodiment includes a CPU, a memory, and a storage device, and allows the CPU to load a program retained in advance in the storage device to the memory for execution, to thereby realize the functions of the sequence control unit 120, the image generating unit 140, the transmission condition setting unit 130, and the elasticity evaluating unit 150. A variety of data used in processes of the respective functions, and a variety of data generated during the processes are stored in the storage device. At least one of the respective functions of the elasticity evaluating unit 150 may be provided in an external information processing apparatus capable of performing data transmission/reception with the diagnostic ultrasound apparatus 100. Further, the entirety or some of the functions of the respective units may be realized by hardware such as an application specific integrated circuit (ASIC) or a field-programmable gate array (FPGA).

<Flow of imaging>

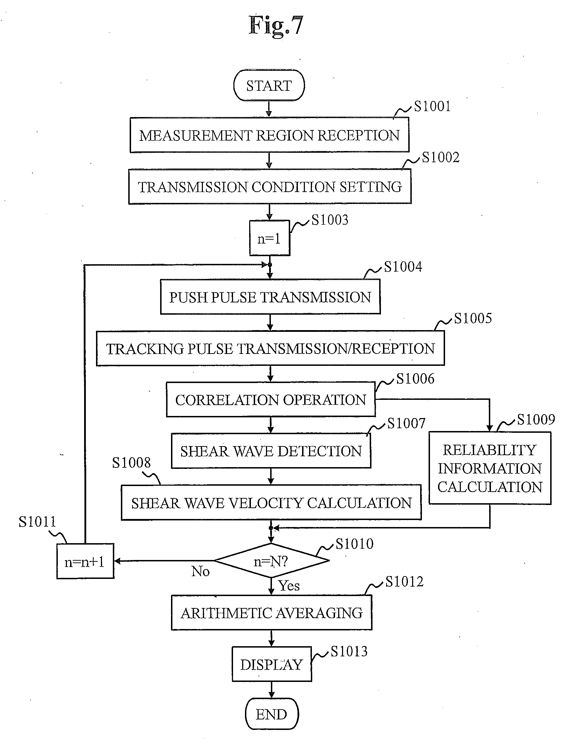

Next, the flow of an imaging process when the radiation pressure elastography is executed by the diagnostic ultrasound apparatus 100 of the embodiment will be described with reference to Fig. 7. This process starts using an instruction from the user as a trigger. Here, it is assumed that push pulses are transmitted N times.

First, an operator designates a measurement region of a shear wave on a B-mode image. The operator designates the measurement region through the input device 170. The transmission condition setting unit 130 receives the designated measurement region (step S1001), and sets transmission conditions of the push pulses and tracking pulses (step S1002).

After the transmission conditions of the push pulses and the tracking pulses are set, the sequence control unit 120 starts a radiation pressure elasticity measurement. Here, first, a counter value n for counting the number of times of measurement is initialized (n=1) (step S1003). Further, the push pulses are transmitted according to the set conditions (step S1004). In addition, immediately after the transmission of the push pulses, transmission of the tracking pulses is started (step S1005).

The sequence control unit 120 converts echo signals obtained by the transmission of the tracking pulses into complex RF data, and the correlation operating section 151 performs a complex correlation operation with respect to the data (step S1006). The complex correlation operation result is input to the shear wave detecting section 152 and the fluctuation evaluating section 154.

The shear wave detecting section 152 calculates a peak position and a peak detection time of a shear wave from the complex correlation operation result, to thereby detect the shear wave (step S1007). Further, the velocity calculating section 153 calculates a shear wave velocity from the peak position and the peak detection time (step S1008). The calculated shear wave velocity is retained in the storage device in association with the number n of times of measurement.

On the other hand, the fluctuation evaluating section 154 calculates reliability information from the complex correlation operation result (step S1009). The calculated reliability information is retained in the storage device in association with the number n of times of measurement.

The sequence control unit 120 decides whether the measurement is performed N times (step S1010). When the measurement is not performed N times, the sequence control unit 120 increments the counter value n by 1 (step S1011), and makes the procedure to return to step S1004 to repeat the processes.

On the other hand, when it is determined in step S1012 that the measurement is performed N times, the arithmetic averaging section 155 calculates an arithmetic average velocity $VS_{mean}$ (step S1012). The arithmetic average may be weighted using a fluctuation index. Here, a standard deviation SD may be calculated together. Further, the fluctuation evaluating section 154 may also calculate a standard deviation value KM(SD) of the reliability information.

The presenting section 156 generates a display screen using the calculation results, displays the display screen on the display device 180 (step S1013), and then, terminates the procedure.

As described above, the diagnostic ultrasound apparatus 100 of the embodiment includes the shear wave detecting section 152 that detects a shear wave generated at a focus position of burst ultrasound by transmitting the burst ultrasound focused on a subject, using an echo signal group obtained by repeatedly transmitting plural shear wave detection pulses, the velocity calculating section 153 that calculates a shear wave velocity which is a propagation velocity of the shear wave, the fluctuation evaluating section 154 that evaluates a fluctuation in a measurement region including a propagation region of the shear wave and obtains the evaluation result as reliability information indicating the reliability of the shear wave velocity, and the presenting section 156 that presents the reliability information to a user.

Here, the fluctuation evaluating section 154 may perform the evaluation of the fluctuation using the echo signal group.

Further, the fluctuation evaluating section 154 specifies a factor of the fluctuation, and the presenting section 156 presents the specified factor to the user.

In this way, according to this embodiment, information relating to a motion is extracted from tracking pulses used in the radiation pressure elastography, and a motion or a surface deviation in a tissue of a measurement target is detected while detecting a shear wave. By providing information relating to the detected motion a user, it is possible to provide a guide relating to the reliability of measurement to the user. Further, in arithmetic averaging, by performing weighting of a shear wave velocity measurement value according to the motion information, it is possible to provide a measurement value with enhanced reliability. In addition, it is detected whether image shaking due to a motion pattern is present, and when the image shaking is present, the fact is notified to the user.

Thus, the user can recognize the reliability of the measurement by the provided information. Further, it is possible to appropriately change the measurement. In this way, according to this embodiment, it is possible to realize a measurement method with enhanced reliability. Thus, according to this embodiment, in the radiation pressure elastography, it is possible to reduce deterioration in measurement accuracy and reproducibility, and to provide an ultrasound image (hardness information) having enhanced diagnostic performance to the user.

According to the radiation pressure elastography, for example, since breast cancer or the like has high hardness compared with peripheral tissues, by drawing a hard portion, it is possible to detect the breast cancer with high sensitivity. Further, in hepatitis or the like that makes cirrhosis of the liver, since the hardness of the liver is strongly related to the progress of the disease, by measuring the hardness of the liver, it is possible to perform precise diagnosis and treatment progress monitoring while suppressing the number of biopsies to the minimum. According to this embodiment, it is possible to maintain the above-described advantages of the radiation pressure elastography, and to obtain the above effects without addition of new measurement.

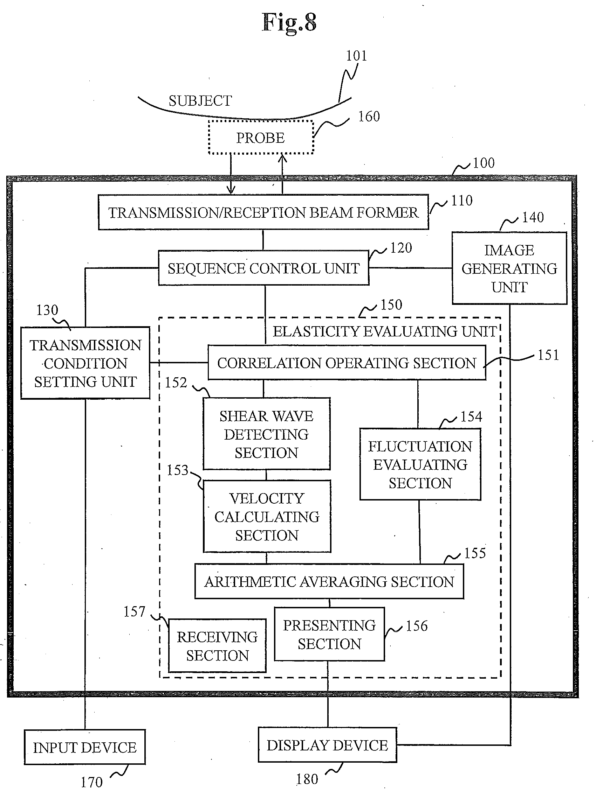

In the above-described embodiment, a configuration in which the presenting section 156 generates a display screen and presents a shear wave velocity and an evaluation result thereof to a user is described, but the invention is not limited thereto. For example, as shown in Fig. 8, in addition to the configuration of the above-described embodiment, a receiving section 157 that receives an instruction from a user through a display screen 600 may be provided. In this case, the display screen 600 generated by the presenting section 156 includes a reception button 630 for receiving an instruction for re-measurement.

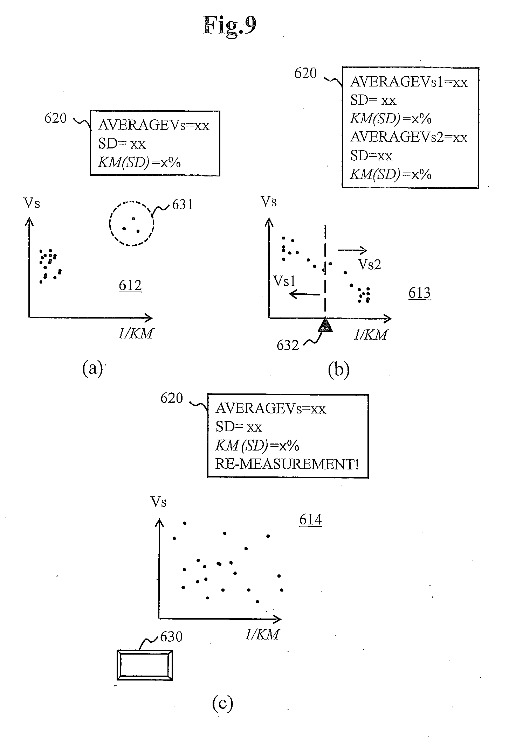

For example, as shown in Fig. 9(a), when the display screen 612 is displayed, a user selects plotted points with respect to a small reciprocal of a fluctuation index KM through the display screen 612. The selection is performed by surrounding the plotted points by a frame 631, as shown in the figure, for example. The receiving section 157 receives the selection, specifies a corresponding shear wave velocity, and makes the arithmetic averaging section 155 to calculate again an arithmetic average velocity only using the selected shear wave velocity. The calculation result is displayed by the presenting section 156.

Further, as shown in Fig. 9(b), when the display screen 613 is displayed, the user divides plotted points into sets by a reciprocal of the fluctuation index KM which is arbitrarily set, for example. Instruction of the division is performed, for example, by designating a reciprocal 632 of a predetermined fluctuation index KM on a scatter plot of the display screen 613, as shown in the figure. The receiving section 157 receives the designation of the reciprocal of the fluctuation index KM used in the division, and groups the sets of the plotted points divided by a value of the reciprocal, and makes the arithmetic averaging section 155 to calculate an arithmetic average velocity for each group. The calculation result is displayed by the presenting section 156.

Further, as shown in Fig. 9(c), when the display screen 614 is displayed, the user instructs re-measurement through the reception button 630, for example. If the instruction is received, the receiving section 157 instructs the sequence control unit 120 to perform the measurement again. Here, an instruction for changing the grip of the probe 160 may also be given.

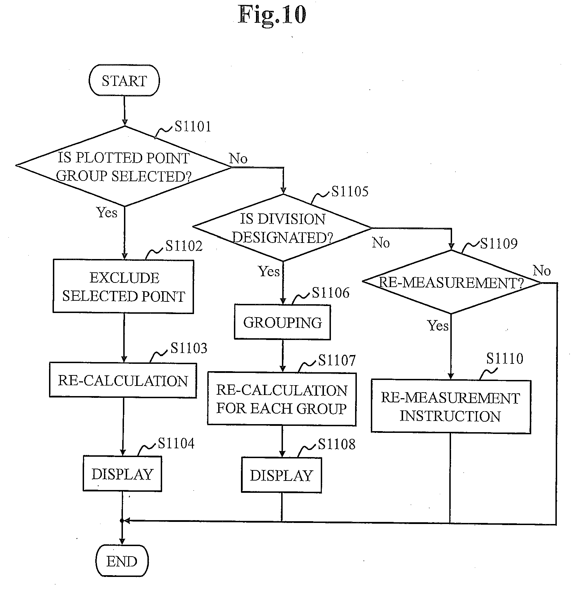

The flow of processes after the display in this case will be described with reference to Fig. 10.

When a predetermined plotted point group is selected through the display screen 600 (step S1101), the receiving section 157 excludes shear wave velocity data corresponding to the plotted point group (step S1102). Further, the receiving section 157 makes the arithmetic averaging section 155 to calculate an arithmetic average again based on the remaining shear wave velocity data (step S1103). Here, the fluctuation evaluating section 154 may calculate a standard deviation of fluctuation indexes KM of the remaining shear velocity data. Further, the presenting section 156 generates a display screen from the calculation result, and displays the result on the display device 180 (step S1104), and then, terminates the process.

On the other hand, when the receiving section 157 receives designation of a reciprocal of the fluctuation index KM for dividing the plotted point group through the display screen 600 (step S1105), the receisving sevtion 157 divides and groups the plotted points into plotted points which are equal to or greater than the reciprocal of the fluctuation index KM and plotted points which are smaller than the reciprocal (step S1105). Then, the receiving section 157 makes the arithmetic averaging section 155 to calculate the arithmetic average again using the shear wave velocity data for each group (step S1106). Here, the fluctuation evaluating section 154 may calculate a standard deviation of the fluctuation indexes KM of the shear wave velocity data for each group. Further, the presenting section 156 generates a display screen of each group from the calculation result, displays the result on the display device 180 (step S1107), and then, terminates the process.

Further, when pressing of an instruction button for instructing re-calculation is received (step S1108), the receiving section 157 instructs the sequence control unit 120 to perform re-measurement (step S1109). When there is no instruction, the process is terminated.

As described above, since the receiving section 157 is provided and an instruction from a user is received based on reliability information, it is possible to feed back a reliability evaluation result for measurement, and to enhance measurement accuracy and reproducibility.

[REFERENCE SIGNS LIST]

100    DIAGNOSTIC ULTRASOUND APPARATUS
110    TRANSMISSION/RECEPTION BEAM FORMER
120    SEQUENCE CONTROL UNIT
130    TRANSMISSION CONDITION SETTING UNIT
140    IMAGE GENERATING UNIT
150    ELASTICITY EVALUATING UNIT

151 CORRELATION OPERATING SECTION
152 SHEAR WAVE DETECTING SECTION
153 VELOCITY CALCULATING SECTION
154 FLUCTUATION EVALUATING SECTION
155 ARITHMETIC AVERAGING SECTION
156 PRESENTING SECTION
157 RECEIVING SECTION
160 PROBE
170 INPUT DEVICE
180 DISPLAY DEVICE
201 MEASUREMENT REGION
210 B-MODE IMAGE
211 TISSUE LAYER
212 TISSUE LAYER
213 TISSUE LAYER
220 MEASUREMENT REGION
221 SHEAR WAVE
222 SHEAR WAVE GENERATING POSITION
231 NON-PROPAGATION REGION
232 SHEAR WAVE PROPAGATION REGION
233 NON-PROPAGATION REGION
234 ARROW
241 CHANGE OF CORRELATION COEFFICIENT
242 CHANGE OF CORRELATION COEFFICIENT
243 CHANGE OF CORRELATION COEFFICIENT
310 B-MODE IMAGE
311 SUPERFICIAL TISSUE
312 LIVER
313 ANOTHER TISSUE
314 ARROW
320 MEASUREMENT REGION
340 CHANGE PATTERN OF CORRELATION COEFFICIENT
350 CHANGE PATTERN OF CORRELATION COEFFICIENT
360 CHANGE PATTERN OF CORRELATION COEFFICIENT
600 DISPLAY SCREEN
610 SCATTER PLOT
611 DISPLAY SCREEN
612 DISPLAY SCREEN
613 DISPLAY SCREEN
614 DISPLAY SCREEN
620 REFERENCE INFORMATION
630 RECEPTION BUTTON
631 FRAME
632 DESIGNATED POSITION

**Claims**

1.  A diagnostic ultrasound apparatus comprising:

    a shear wave detecting section that detects a shear wave generated at a focusing position of a burst ultrasonic wave by transmitting the focused burst ultrasonic wave to a subject using an echo signal group obtained by repeatedly transmitting a plurality of shear wave detection pulses;
    a velocity calculating section that calculates a shear wave velocity which is a propagation velocity of the shear wave;
    a fluctuation evaluating section that evaluates a fluctuation in a measurement region including a propagation region of the shear wave and obtains a evaluation result as reliability information indicating reliability of the shear wave velocity; and

a presenting section that presents the reliability information to a user.

2. The diagnostic ultrasound apparatus according to claim 1,
wherein the fluctuation evaluating section obtains the reliability information based on a fluctuation in a depth region which is present within the measurement region and has a depth different from the depth of the propagation region of the shear wave.

3. The diagnostic ultrasound apparatus according to claim 1,
wherein the reliability information is an index indicating a size of the fluctuation.

4. The diagnostic ultrasound apparatus according to claim 1,
wherein the fluctuation evaluating section further specifies a factor of the fluctuation, and
the presenting section further presents the specified factor to the user.

5. The diagnostic ultrasound apparatus according to claim 1, further comprising:

a sequence control unit that executes measurement that includes transmission of the burst ultrasonic wave, repetitive transmission of the plurality of shear wave detection pulses, and reception of an echo signal due to the transmission, according to a predetermined pulse sequence; and
an arithmetic averaging section that calculates an arithmetic average of the plurality of shear wave velocities, wherein the sequence control unit repeats the measurement,
the shear wave detecting section detects the shear wave for each measurement,
the velocity calculating section calculates the shear wave velocity whenever the shear wave is detected,
the arithmetic averaging section calculates an arithmetic average of the plurality of shear wave velocities calculated whenever the shear wave is detected, and
the presenting section presents the arithmetic average result together with the reliability information to the user.

6. The diagnostic ultrasound apparatus according to claim 5,
wherein the arithmetic averaging section performs weighting using the reliability information in calculating the arithmetic average.

7. The diagnostic ultrasound apparatus according to claim 5,
wherein the presenting section further presents the reliability information and the shear wave velocity for each measurement as a scatter plot.

8. The diagnostic ultrasound apparatus according to claim 7, further comprising:

a receiving section that receives an instruction from the user through a plotted result on the scatter plot,
wherein the arithmetic averaging section calculates the arithmetic average again according to the instruction.

9. The diagnostic ultrasound apparatus according to claim 8,
wherein the receiving section receives selection of the shear wave velocity to be excluded, and
the arithmetic averaging section calculates the arithmetic average again using shear wave velocities other than the selected shear wave velocity.

10. The diagnostic ultrasound apparatus according to claim 8,
wherein the receiving section receives an instruction for dividing the shear wave velocities into a plurality of groups according to the reliability information, and
the arithmetic averaging section calculates an arithmetic average of the shear wave velocities for each group again.

11. The diagnostic ultrasound apparatus according to claim 5, further comprising:

a receiving section that receives an instruction for re-measurement from the user,
wherein the sequence control unit executes the measurement according to the instruction.

12. The diagnostic ultrasound apparatus according to claim 2,
wherein the fluctuation evaluating section specifies a depth region where the shear wave propagates based on a position where the shear wave is generated and an amplitude of the shear wave.

**13.** The diagnostic ultrasound apparatus according to claim 2,
wherein the fluctuation evaluating section specifies a depth region where the shear wave propagates using a correlation coefficient obtained by performing a correlation operation in a time direction with respect to data obtained from the echo signal group.

**14.** The diagnostic ultrasound apparatus according to claim 4,
wherein the fluctuation evaluating section specifies the factor using a change pattern of a correlation coefficient obtained by performing a correlation operation in a time direction with respect to data obtained from the echo signal group.

**15.** An elasticity evaluation method comprising:

detecting a shear wave generated at a focusing position of a burst ultrasonic wave by transmitting the burst ultrasonic wave focused on a subject using an echo signal group obtained by repeatedly transmitting a plurality of shear wave detection pulses;
calculating a shear wave velocity which is a propagation velocity of the shear wave;
evaluating a fluctuation in a measurement region including a propagation region of the shear wave and obtaining a evaluation result as reliability information indicating reliability of the shear wave velocity; and
presenting the reliability information to a user.

# Fig.1

SUBJECT — 101

PROBE — 160

100

```
TRANSMISSION/RECEPTION BEAM FORMER — 110
```

IMAGE GENERATING UNIT — 140

SEQUENCE CONTROL UNIT — 120

150

TRANSMISSION CONDITION SETTING UNIT — 130

ELASTICITY EVALUATING UNIT — 150

CORRELATION OPERATING SECTION — 151

SHEAR WAVE DETECTING SECTION — 152

FLUCTUATION EVALUATING SECTION — 154

VELOCITY CALCULATING SECTION — 153

ARITHMETIC AVERAGING SECTION — 155

PRESENTING SECTION — 156

INPUT DEVICE — 170

DISPLAY DEVICE — 180

# Fig.2

(a)

(b)

# Fig.3

# Fig.4

(a)

(b)

(c)

(d)

Fig.5

Vs

620 〜 AVERAGE Vs=xx
SD= xx
*KM(SD)*=x%

〜 610

1/KM

〜630

600

# Fig.6

620 ⟍ 

AVERAGEVs=xx
SD= xx
KM(SD)=x%

620 ⟍ 

AVERAGEVs=xx
SD= xx
KM(SD)=x%

611

Vs

1/KM

(a)

612

Vs

1/KM

(b)

620 ⟍ 

AVERAGEVs=xx
SD= xx
KM(SD)=x%

620 ⟍ 

AVERAGEVs=xx
SD= xx
KM(SD)=x%
RE-MEASUREMENT!

613

Vs

1/KM

(c)

614

Vs

1/KM

(d)

# Fig.7

```
            ( START )
                |
                |  ~S1001
   ┌────────────────────────────┐
   │ MEASUREMENT REGION RECEPTION │
   └────────────────────────────┘
                |  ~S1002
   ┌────────────────────────────┐
   │ TRANSMISSION CONDITION SETTING │
   └────────────────────────────┘
                |  ~S1003
            ┌───────┐
            │  n=1  │
            └───────┘
                |
                ↓
        ┌──────────────────┐
        │ PUSH PULSE TRANSMISSION │  ~S1004
        └──────────────────┘
                |  ~S1005
   ┌─────────────────────────────────────┐
   │ TRACKING PULSE TRANSMISSION/RECEPTION │
   └─────────────────────────────────────┘
                |  ~S1006
        ┌──────────────────┐
        │ CORRELATION OPERATION │────────────┐
        └──────────────────┘                 |  ~S1009
                |  ~S1007               ┌──────────────┐
        ┌──────────────────┐           │ RELIABILITY  │
        │ SHEAR WAVE DETECTION │       │ INFORMATION  │
        └──────────────────┘           │ CALCULATION  │
            S1008   |                   └──────────────┘
   ┌─────────────────────────────────┐       |
   │ SHEAR WAVE VELOCITY CALCULATION │       |
   └─────────────────────────────────┘       |
   S1011        |                             |
  ┌───────┐     |   ~S1010                    |
  │ n=n+1 │◄────◇  n=N?  ◄────────────────────┘
  └───────┘  No  ◇
                |  Yes  ~S1012
        ┌──────────────────┐
        │ ARITHMETIC AVERAGING │
        └──────────────────┘
                |  ~S1013
            ┌─────────┐
            │ DISPLAY │
            └─────────┘
                |
             ( END )
```

# Fig.8

101

SUBJECT

160

PROBE

100

TRANSMISSION/RECEPTION BEAM FORMER ~ 110

140

IMAGE GENERATING UNIT

SEQUENCE CONTROL UNIT ~ 120

130

TRANSMISSION CONDITION SETTING UNIT

150

ELASTICITY EVALUATING UNIT

CORRELATION OPERATING SECTION ~ 151

152

SHEAR WAVE DETECTING SECTION

154

FLUCTUATION EVALUATING SECTION

153

VELOCITY CALCULATING SECTION

155

ARITHMETIC AVERAGING SECTION

157

RECEIVING SECTION

PRESENTING SECTION ~ 156

INPUT DEVICE

170

DISPLAY DEVICE

180

# Fig.9

620 ⌇ 
AVERAGEVs=xx
SD= xx
*KM(SD)=x%*

620 ⌇
AVERAGEVs1=xx
SD= xx
*KM(SD)=x%*
AVERAGEVs2=xx
SD=xx
*KM(SD)=x%*

Vs

~631

612

*1/KM*

(a)

Vs

Vs1    Vs2

613

632 ⌇

*1/KM*

(b)

620 ⌇
AVERAGEVs=xx
SD= xx
*KM (SD)=x%*
RE-MEASUREMENT!

Vs

614

~630

*1/KM*

(c)

# Fig.10

```
        ┌─────────┐
        │  START  │
        └─────────┘
             │
             │    ～S1101
             ▼
      ◇ IS PLOTTED POINT ◇──No──┐
      ◇ GROUP SELECTED? ◇        │
             │                   │
            Yes                  │    ～S1105
             │                   ▼
             │    ～S1102    ◇ IS DIVISION ◇──No──┐
             ▼               ◇ DESIGNATED? ◇       │
      ┌─────────────┐            │                 │  ～S1109
      │   EXCLUDE   │           Yes                ▼
      │SELECTED POINT│           │    ～S1106  ◇RE-MEASUREMENT?◇──No──┐
      └─────────────┘            ▼            ◇              ◇        │
             │            ┌────────────┐         │                    │
             │  ～S1103   │  GROUPING  │        Yes                   │
             ▼            └────────────┘         │    S1110           │
      ┌─────────────┐            │    ～S1107    ▼      ～            │
      │RE-CALCULATION│           ▼          ┌──────────────┐          │
      └─────────────┘    ┌──────────────┐   │RE-MEASUREMENT│          │
             │           │RE-CALCULATION│   │ INSTRUCTION  │          │
             │  ～S1104  │FOR EACH GROUP│   └──────────────┘          │
             ▼           └──────────────┘         │                  │
      ┌─────────────┐           │    ～S1108       │                  │
      │   DISPLAY   │           ▼                  │                  │
      └─────────────┘    ┌──────────────┐          │                  │
             │           │   DISPLAY    │          │                  │
             │           └──────────────┘          │                  │
             │                  │                  │                  │
             ├──────────────────┴──────────────────┴──────────────────┘
             ▼
        ┌─────────┐
        │   END   │
        └─────────┘
```

## INTERNATIONAL SEARCH REPORT

| | International application No. |
|---|---|
| | PCT/JP2014/069484 |

| A. CLASSIFICATION OF SUBJECT MATTER |
|---|
| *A61B8/08*(2006.01)i |

According to International Patent Classification (IPC) or to both national classification and IPC

| B. FIELDS SEARCHED |
|---|
| Minimum documentation searched (classification system followed by classification symbols) |
| A61B8/08 |

Documentation searched other than minimum documentation to the extent that such documents are included in the fields searched

| Jitsuyo Shinan Koho | 1922–1996 | Jitsuyo Shinan Toroku Koho | 1996–2014 |
| Kokai Jitsuyo Shinan Koho | 1971–2014 | Toroku Jitsuyo Shinan Koho | 1994–2014 |

Electronic data base consulted during the international search (name of data base and, where practicable, search terms used)

| C. DOCUMENTS CONSIDERED TO BE RELEVANT |
|---|

| Category* | Citation of document, with indication, where appropriate, of the relevant passages | Relevant to claim No. |
|---|---|---|
| X<br>Y<br>A | WO 2011/004661 A1 (Hitachi Medical Corp.),<br>13 January 2011 (13.01.2011),<br>paragraphs [0025] to [0075]; fig. 1 to 9<br>& JP 2010-20962 A | 1-3,12,13,15<br>5-8,11<br>4,9,10,14 |
| Y<br>A | WO 2012/135611 A2 (HITACHI ALOKA MEDICAL INC.),<br>04 October 2012 (04.10.2012),<br>page 20, lines 7 to 12; fig. 12<br>& US 2012/0253194 A1 & EP 2691026 A<br>& CN 103458800 A & JP 2014-512217 A | 5-8,11<br>4,9,10,14 |

☐ Further documents are listed in the continuation of Box C.  ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 30 September, 2014 (30.09.14) | 07 October, 2014 (07.10.14) |

| Name and mailing address of the ISA/ | Authorized officer |
|---|---|
| Japanese Patent Office | |
| Facsimile No. | Telephone No. |

Form PCT/ISA/210 (second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20100016718 A